# FASCICULE DE BREVET EUROPEEN

(11) **EP 1 029 073 B1**
(45) Date de publication et mention de la délivrance du brevet: **09.05.2007**
(21) Numéro de dépôt: 98954534.8
(22) Date de dépôt: 06.11.1998
(51) Int. Cl.: C12Q 1/04, C12Q 1/34, C07C 311/19, C07D 233/54

(54) **PROCEDE ET AGENT DE DETERMINATION D'UNE ACTIVITE ENZYMATIQUE DE TYPE DESAMINASE**
VERFAHREN UND AGENS ZUR BESTIMMUNG VON DEAMINASE-AKTIVITÄT
METHOD AND AGENT FOR DETERMINING DEAMINASE ACTIVITY

(30) Priorité: 06.11.1997 FR 9714191
(43) Date de publication de la demande: 23.08.2000
(73) Titulaire: BIO MERIEUX, 69280 Marcy l'Etoile (FR)
(72) Inventeur: ARMSTRONG, Lyle, Newcastle upon Tyne (GB); JAMES, Arthur, Newcastle upon Tyne NE2 1HR (GB); ORENGA, Sylvain, F-01160 Neuville sur Ain (FR)
(74) Mandataire: Guerre, Dominique
(86) Numéro de dépôt international: PCT/FR1998/002380
(87) Numéro de publication internationale: WO 1999/024604

(56) Documents cités:
- WO-A-92/00068
- US-A- 3 410 896
- US-A- 4 603 108
- US-A- 5 411 867
- US-A- 5 541 082
- US-A- 5 643 743
- GIAMMANCO G. & PIGNATO S.: "Rapid identification of micro-organisms from urinary tract infections by beta-glucuronidase, phenylalanine deaminase, cytochrome oxidase and indole tests on isolation media." JOURNAL OF MEDICAL MICROBIOLOGY, vol. 41, no. 6, décembre 1994, pages 389-392, XP002073584 cité dans la demande
- MANAFI M. & ROTTER M. L.: "A new plate medium for rapid presumptive identification and differentiation of Enterobacteriaceae." INTERNATIONAL JOURNAL OF FOOD MICROBIOLOGY, vol. 14, no. 2, novembre 1991, pages 127-134, XP002073585 cité dans la demande
- CHEMICAL ABSTRACTS, vol. 78, no. 5, 5 février 1973 Columbus, Ohio, US; abstract no. 26194, PELOUX, Y. & LEFORT, H.: "Lysine deaminase of the Proteus - Providencia group by means of the Edwards and Fife lysine-iron medium. Practical value of this medium for differentiating enterobacteria." XP002073586 & FEUILL. BIOL., vol. 13, no. 68, 1972, pages 37-42, cité dans la demande
- CHEMICAL ABSTRACTS, vol. 96, no. 19, 10 mai 1982 Columbus, Ohio, US; abstract no. 158923, SIVOLODSKII, E. P.: "Modification of a method for the determination of tryptophan deaminase and phenylalanine deaminase content in bacteria." XP002073587 & LAB. DELO, no. 3, 1982, pages 166-168, cité dans la demande
- NOBUTAKA FUJI, ET. AL.: CHEMICAL PHARMACEUTICAL BULLETIN, vol. 32, no. 7, 1984, pages 2660-2665, XP001194788
- P.G. KATSOYANNIS, ET AL.: CONTRIBUTIONS FROM THE DEPARTMENT OF BIOCHEMISTRY, CORNELL UNIVERSITY MEDICAL COLLEGE, vol. 79, 1957, pages 4516-4520,
- SHUMPEI SAKAKIBARA AND TOSHIYUKI FUJI: BULLETIN OF CHEMICAL SOCIETY OF JAPAN, vol. 42, no. 5, 1969, page 1466,

## Description

La présente invention concerne un procédé de détection et d'identification et/ou quantification d'une activité enzymatique de type désaminase dans un milieu de culture pour micro-organismes, et un agent de détection adaptés à ce procédé, le procédé de préparation de ces composés et agents de détection, ainsi que les milieux de culture pour mettre en oeuvre ledit procédé.

La détection et l'identification des micro-organismes sont très importantes notamment en médecine, dans l'industrie agro-alimentaire, pour le contrôle de l'environnement (eau...). Les micro-organismes peuvent être recherchés pour leur pathogénicité, comme indicateurs de contamination, pour le suivi de procédés de fabrication et autre.

Les techniques de détection et d'identification des micro-organismes sont actuellement basées sur la recherche de séquences nucléotidiques caractéristiques, la recherche d'antigènes ou d'anticorps, la culture en milieu sélectif ou non, ou encore sur la recherche d'activités métaboliques et notamment enzymatiques (par exemple activités osidases, estérases, peptidases, oxydases, etc...).

Le plus souvent les procédés de détection et d'identification et/ou quantification des micro-organismes associent plusieurs de ces techniques. La culture est ainsi utilisée pour multiplier et sélectionner les micro-organismes recherchés. Afin de simplifier leur détection, il a été proposé de mettre en évidence des activités biochimiques en introduisant des molécules produisant une coloration ou une fluorescence, directement dans le milieu de culture. De tels milieux sont appelés milieux de détection. Les activités biochimiques peuvent être mises en évidence par diverses méthodes telles que :
- la modification physico-chimique du milieu : changement de pH en présence d'un indicateur coloré, ou fluorescent (méthyl-umbelliférone,...),
- le changement du potentiel rédox révélé à l'aide d'un indicateur coloré (sels de tétrazolium,...) ou fluorescent (EP-A-0 424 293),
- l'hydrolyse de molécules libérant un composé coloré ou fluorescent (indoxyle, naphtol, coumarine...),
- la réaction d'une molécule produite par les micro-organismes avec un composé présent dans le milieu et résultant en une coloration (détection d'indole, James *et al*., 1986).

On sait que les milieux gélifiés (ou solides) sont particulièrement bien adaptés à la culture et à l'isolement des micro-organismes à partir d'un prélèvement, ainsi qu'à la détection de micro-organismes "cibles" au sein d'un mélange de taxa de micro-organismes.

On connaît un procédé de différenciation des bactéries du groupe *Proteus* des autres entérobactéries (Sverre Dick Henriksen, State Institute for Public Health, Bacteriological Department, Oslo, Norway, 6 juin 1950). Ce procédé met en oeuvre une quantité égale, en milieu gélosé, de D- et L-phénylalanine en présence de sel de fer. La réaction colorée verte obtenue, en comparaison avec le test de l'uréase, bien que positive pour identifier le groupe *Proteus,* est présentée comme étant un test lourd à mettre en oeuvre.

On connaît encore le document de GIAMMANCO & PIGNATO (" Rapid identification of micro-organisms from urinary tract infections by beta-glucuronidase, phenylalanine deaminase, cytochrome oxidase and indole tests on isolation media ", JOURNAL OF MEDICAL MICROBIOLOGY, vol.41 , No 6, décembre 1994, pages 389-392) qui divulgue, pour la détection d'une activité désaminase des bactéries du groupe des Proteeae, l'utilisation des acides aminés naturels tels que le tryptophane et la phénylalanine, en combinaison avec un sel de fer (FeCl₃).

On connaît encore Le document de MANAFI & ROTTER (" A new plate medium for rapid presumptive identification and differentiation of Enterobacteriaceae ", INTERNATIONAL JOURNAL OF FOOD MICROBIOLOGY, vol. 14, N°2, novembre 1991, pages 127-134) qui divulgue, pour la détection d'une activité désaminase des bactéries du groupe des Proteeae, l'utilisation du tryptophane seul avec du sel de fer (citrate de fer ammoniacal). Ce document mentionne, pour d'autres marqueurs d'activité enzymatique (4-méthylumbélliférone, nitrophényl), qu'une diffusion de ces marqueurs est pénalisante. La seule solution pour en minimiser l'effet est d'incuber les milieux pendant un temps réduit.

On connaît encore le document de PELOUX & LEFORT (" Lysine deaminase of the Proteus-Providencia group by means of the Edwards and Fife lysine-iron medium. Practical value of this medium for differentiating enterobacteria ", FEUILL.BIOL., vol. 13, N°68, 1972, pages 37-42) qui compare l'activité de trois acides-aminés (tryptophane, phénylalanine et lysine) pour la détection de la désaminase en association avec un sel de fer.

On connaît encore le document de SIVOLODSKII (" Modification of a method for the determination of tryptophan deaminase and phenylalanine deaminase content in bacteria ", LAB. DELO., N°3, 1982, pages 166-168) qui propose d'ajouter des hydrolysats enzymatiques de protéines, constitués de mélanges d'acides aminés naturels, de peptides et d'autres composés, pour détecter des activités tryptophane et phénylalanine désaminases.

On connaît encore la demande de brevet WO-A-92/00068, qui divulgue des composés histidine substitués agissant en tant qu'antagonistes des récepteurs de l'angiotensine II. Ces composés, selon la formule (I) et les exemples, comprennent entre autre la substitution du groupe amine ou carboxyle.

On connaît encore les brevets US-A-5,643,743 et US-A-5,411,867 qui divulguent le tryptophane pour la détection de la tryptophanase, enzyme différente des désaminases.

On connaît encore le brevet US-A-4,603,108 qui divulgue comme substrat pour détecter la phénylalanine désaminase, la D,L-béta-(p-nitrophényl)-alanine. La lecture de la réaction est effectuée soit à 480 nm, sans ajout de révélateur pour la phénylalanine désaminase, soit par dosage de l'ammoniac pour la leucine désaminase.

On connaît enfin le brevet US-A-5,541,082 qui divulgue, pour la détection des désaminases, les acides aminés phénylalanine et tryptophane, qui permettent d'obtenir une couleur orangée qui diffuse dans le milieu.

Ces documents divulguent l'utilisation d'acides aminés naturels, ce qui est incompatible avec une limitation de la diffusion de l'alpha-kéto acide. La seule solution proposée par certains de ces documents revient à limiter le temps d'incubation, ce qui est très préjudiciable à la qualité de la détection. Or la limitation de la diffusion est un gage de différentiation de plusieurs colonies microbiennes présentes dans un même milieu. Soit ces documents divulguent l'utilisation d'acides aminés artificiels et modifiés qui ne répondent pas à la formule (1) de l'invention, et qui peuvent, en plus, proposer une application différente, comme par exemple l'antagonisme avec les récepteurs de l'angiotensine II.

Malgré tous les tests biochimiques actuellement sur le marché, il s'avère qu'on ne dispose pas actuellement de moyens particulièrement bien adaptés et faciles à mettre en oeuvre, notamment en milieu gélosé, pour détecter et identifier et/ou quantifier dans une culture plurimicrobienne une activité enzymatique de type désaminase de micro-organismes.

La présente invention se propose donc de résoudre ce problème.

Un premier objet de l'invention est un procédé de détection et d'identification et/ou quantification d'une activité enzymatique de type désaminase de micro-organisme, selon lequel on met en contact un inoculum susceptible de contenir un micro-organisme ayant une activité désaminase avec un milieu de culture pour micro-organismes,
le milieu de culture comprenant au moins un agent de détection permettant de mettre en évidence, par formation d'un produit coloré avec un agent révélateur, une activité enzymatique de type désaminase,
ledit agent de détection est un L-acide aminé cyclique de formule (1) générale suivante: 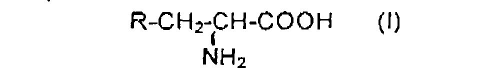 dans laquelle:
- R représente un radical d'acide-aminé cyclique, substitué par 1 à 3 groupements X, identiques ou différents,
- X représente un groupement limitant la diffusion de l'α-kéto acide produit par la désamination de l'acide-aminé cyclique, et est choisi parmi le méthyle, le benzyle, le carboxybenzle, le dansyle, le naphtaline - sulfonyle, le rosyl-sulfonyle, le mésitylène-sulfonyle.
le composé de formule (I) pouvant être substitué par différents groupements n'interférant pas avec la fonction du groupement X.

On entend par " groupement limitant la diffusion ":
- tout groupement de type hydrophobe limitant la diffusion dans un milieu hydrophile, ou
- tout groupement permettant de s'associer, par des liaisons faibles, notamment hydrophobes, ou de se lier, par une ou des liaisons covalentes, notamment thiol, aux constituants des cellules des micro-organismes, tels que la paroi, la membrane, les protéines .......

A titre de test définissant les groupements limitant la diffusion, on inocule en spot au centre de deux boîtes de pétri respectivement un L-acide-aminé cyclique et un agent de détection correspondant selon l'invention. On mesure les diamètres de la diffusion des α-céto-acides produits par l'activité enzymatique de type désaminase de l'inocculat. Le diamètre de diffusion de l'agent de détection selon l'invention doit être inférieur à celui du L-acide, aminé cyclique, il sera alors considéré que l'agent de détection limite la diffusion de l'a-céto-acide produit par la désamination du L-acide-aminé.

On entend par « Radical R d'acide aminé cyclique » les radicaux R cycliques ou hétérocycliques tels que indole, phényle, hydroxy-phényle et imidazole, pouvant subir une réaction de substitution par le groupement X. Ces acides-aminés comprennent les acides-aminés naturels ou synthétiques, ou modifiés, notamment par substitution.

On entend par "groupement n'interférant pas avec la fonction du groupement X ", tout groupement qui n'empêche pas le groupement X de limiter la diffusion de l'a-céto-acide.

Dans le procédé selon l'invention, l'ajout d'au moins un agent de détection dans des concentrations adaptées n'inhibe pas la multiplication des micro-organismes dans les milieux de culture appropriés. On peut donc utiliser l'agent de détection en l'ajoutant au milieu de culture des micro-organismes avant le début de la culture ou en début de culture.

L'un des avantages importants du procédé utilisant des agents de détection selon l'invention est qu'en présence d'une activité enzymatique de type désaminase, il donne, après ajout d'un agent révélateur, des produits colorés qui ne diffusent pas dans le milieu, notamment dans un milieu gélifié.

Le procédé selon l'invention peut donc avantageusement être utilisé en milieu gélifié. Il peut aussi, bien entendu, être utilisé en milieu liquide ou sur support solide en l'absence de gélifiant.

Le procédé selon l'invention permet, en milieu plurimicrobien, de détecter et identifier, voir même quantifier les micro-organismes ayant une activité enzymatique de type désaminase, ceci sans interférer avec la détection d'autres micro-organismes. Au contraire, le procédé selon l'invention, permet, par le jeu des réactions enzymatiques produites, de révéler de manière distincte, par des couleurs définissant le type de micro-organisme, le ou les micro-organismes présents dans le milieu.

Le procédé selon l'invention apporte donc l'avantage, outre de détecter et d'identifier une activité enzymatique de type désaminase dans un milieu de culture, de permettre une mise en oeuvre simple et peu coûteuse.

Dans un mode de réalisation préférentiel selon l'invention, l'agent révélateur est un sel de cation.

Le sel de cation, permettant de mettre en évidence l'agent de détection par formation d'un produit coloré, peut être ajouté au milieu de culture en même temps que l'agent de détection, ou bien après la mise en culture des micro-organismes. En effet, l'homme du métier saura déterminer, selon le milieu de culture utilisé, si il est plus avantageux, ou si il est préférable d'ajouter le sel de cation en même temps que l'agent de détection, ou après la mise en culture des micro-organismes. On préfère toutefois, notamment en milieu gélosé, ajouter l'agent révélateur au milieu de culture en même temps que l'agent de détection.

La réaction qui s'effectue entre l'agent de détection et la désaminase, puis l'agent de révélation, en présence de flavoprotéine, lorsqu'on utilise par exemple comme agent de détection la phénylalanine, est la réaction générale suivante :

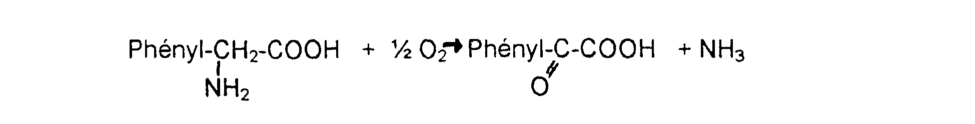

L'acide phénylpyruvique obtenu, qui est un alpha-kéto acide, s'associe avec le chlorure de fer agissant comme agent chélatant, pour donner une coloration verte. Ainsi, quant un tube contenant une telle composition est exposé à l'oxygène atmosphérique, la densité de coloration augmente.

Selon la nature de l'acide aminé utilisé, la couleur finale obtenue varie. Par exemple, dans le cas de l'histidine, la coloration est brune.

Cette réaction est respectée dans le cas où le groupement phényle est remplacé par un autre cycle et est substitué une à trois fois par un groupement X selon la formule (I).

Les micro-organismes détectés et identifiés et/ou quantifiés par l'activité enzymatique de type désaminase selon le procédé de l'invention appartiennent notamment au groupe *Proteus.*

Dans un mode de réalisation très préférentiel selon l'invention, on ajoute également audit milieu de culture au moins un autre agent de détection permettant de mettre en évidence, par formation d'un produit coloré ou fluorescent, une activité enzymatique différente de celle mise en évidence par le composé de formule générale (I). Il peut s'agir par exemple d'une activité estérase, osidase ou peptidase. On peut ainsi obtenir des informations supplémentaires, en liaison avec une absence de coloration (ou de fluorescence) ou en liaison avec une coloration modifiée par rapport à la coloration obtenue avec un seul substrat enzymatique. L'autre agent de détection de formule (I) selon l'invention. Par exemple, on choisira un autre agent de détection capable de donner un produit réactionnel ayant une couleur différente de la couleur obtenue avec les agents de détection de formule (I). L'autre agent de détection (ou second agent de détection) permettra donc de révéler, grâce à sa couleur propre, ou grâce à sa fluorescence, la présence d'une activité enzymatique dont il est spécifique. Si l'activité enzymatique de type désaminase détectable par les agents de détection de formule (I) selon l'invention est également présente, et révélable par une couleur caractéristique, on obtiendra une coloration modifiée, différente de ladite couleur caractéristique et différente aussi de ladite couleur propre donnée par le second agent de détection. Des exemples d'utilisation de plusieurs agents de détection dans le même milieu de culture sont donnés dans les exemples qui suivent. Bien entendu, les résultats peuvent varier avec d'une part le choix de l'agent de détection selon l'invention que l'on utilise, le second (ou plus) agent de détection utilisé et les différents micro-organismes présents dans le milieu de culture.

Les autres agents de détection servant à mettre en évidence des activités enzymatiques différentes sont notamment, mais non exclusivement, des dérivés d'indoxyle, de coumarine, de résorufine, de naphtol, de naphtylamine, de nitrophénol, de nitroaniline, de rhodamine, d'hydroxyquinoléine, de fluorescéine, etc...

Parmi ces autres agents de détection qui peuvent être utilisés en association avec les agents de détection selon l'invention, on peut citer notamment le 5-bromo-4-chloro-3-indolyl-β-D-glururonide, le 6-chloro-3-indolyl-β-D-glucoside, la L-alanine-7-amino-4-méthyl-coumarine, le 4-méthyl-umbelliféryl-N-acétyl-β-D-galactosami-nide, le résorufine-β-D-galactoside, le sulfate de β-naphtyle, le naphtol AS-BI β-D-galactoside, le L-alanine β-naphtylamide, l'O-nitrophénol-β-D-galactoside, le carboxybenzoyle-L-arginine-p-nitroanilide, la rhodamine 110 bis-(L-leucine amide), et le diacétate de fluorescéine.

Un second objet de l'invention est l'utilisation de composés ayant la formule générale (1) suivante; 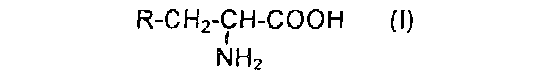 dans laquelle:
- R représente un radical d'acide-aminé cyclique, substitué par un groupement X, [] Choisi parmi le méthyle, le benzyle, le carboxybenzoyle, le dansyle, le naphtaline-sylfonyle, le toluène sulfonyle, le mésitylène-sulfonyle, pour détecter et identifier et/ou quantifier une activité enzymatique de type désaminase de micro-organisme
Les abréviations « N-im » pour un composé de L-histidine, et « N-im » pour un composé de L-tryptophane, signifient que l'atome d'azote du noyau imidazole (His) ou de l'atome d'azote du noyau indole (Trp) est substitué.

Un troisième objet selon l'invention est un agent de détection consistant en le O-(2-naphtalène- sulfonyl)-tyrosine.

On préfère encore plus particulièrement que X soit choisi parmi le naphtalène-sulfonyle, le toluène-sulfonyle, le N-ind-mésitylène-sulfonyle.

Parmi les composés synthétisés, les meilleurs résultats ont été obtenus avec le O-naphtalène-sulfonyl-L-tyrosine, le O-toluène-sulfonyl-L-tyrosine et le N-ind-mésitylène-2-sulphonyl-L-tryptophane. Grâce à ces composés, il a été possible de développer un milieu de culture gélosé permettant de détecter une colonie exprimant une activité enzymatique de type désaminase, y compris au sein de culture pluri-microbienne. De plus, la coloration brune obtenue par ces composés, reste au voisinage de la colonie désaminase⁺, et n'affecte pas l'aspect des colonies désaminase- même proches.

Comme exemple d'agent de détection particulièrement préférés en milieu gélosé, on peut citer:
- le O-naphtalène-sulfonyl-tyrosine.
- le 4-O-toluène-sulfonyl-L-tyrosine.
- le N-toluène-sulfonyle-L-histidine.

On préfère particulièrement également, en milieu liquide, utiliser le N-toluène-sulfonyl-L-histidine.

Un quatrième objet selon l'invention est le procédé de préparation des composés et des agents de détection selon l'invention de formule générale (I) telle que définie respectivement précédemment, comprenant les étapes suivantes:
(a) - formylation du reste R,
(b) - addition d'un sel de X sur le reste R formylé selon (a),
(c) - déformylation du reste R substitué selon (b).

Un cinquième objet selon l'invention est un milieu de culture de micro-organismes comprenant, outre les ingrédients nécessaires à la culture desdits micro-organismes, au moins un agent de détection tel que défini précédemment.

Les agents de détection de formule X-L-acide-aminé cyclique sont utilisés à des concentrations pondérales suffisantes pour donner des réactions colorées observables. Ces concentrations peuvent être déterminées par des expériences de routine par l'homme du métier.

Dans un mode de réalisation préférentiel, la concentration pondérale du ou des agents de détection dans le milieu de culture est comprises entre 0,025 et 5 g/l de milieu de culture.

Dans un mode de réalisation très préférentiel, la concentration pondérale du ou des agents de détection dans le milieu de culture est comprises entre 0,1 et 2 g/l, de préférence entre 0,3 et 0,6 g/l.

Dans un autre mode de réalisation préférentiel, le milieu de culture comprend également un agent révélateur, de préférence un sel de cation, par exemple du citrate de fer ammoniacal. La concentration de l'agent révélateur n'est pas limitante, elle peut être inférieure, égale ou supérieure à celle de l'agent de détection de formule (I).

Dans un autre mode préférentiel de réalisation selon l'invention, le milieu de culture est sous forme gélosé.

Dans un mode de réalisation très préférentiel selon l'invention, le milieu de culture comprend également au moins un autre agent de détection permettant de mettre en évidence, par formation d'un produit coloré ou fluorescent, une activité enzymatique différente de celle mise en évidence par le composé de formule générale (1).

Comme exemple de milieu gélosé, on peut incorporer du O-toluène-sulfonyl-L-tyrosine à 0,5 g/l et du citrate de fer à 0,5 g/l au milieu de culture, dans le milieu suivant :

| | |
|---|---|
| Extrait coeur-cervelle | 5 g/l |
| bio-Soyase | 5 g/l |
| Tampon Tris | 1 g/l |
| Phosphate monopotassique | 1 g/l |
| Agar | 17,5 g/l, |

milieu que l'on peut inocculer avec différents micro-organismes à Gram négatif ou à Gram positif. Seules les souches appartenant au groupe *Proteus* forment des colonies brunes après 18 à 24 heures d'incubation. Les souches n'appartenant pas au groupe *Proteus* forment des colonies qui conservent l'aspect qu'elles ont sur le même milieu dépourvu de O-toluène-sulfonyl-L-tyrosine.

Les caractéristiques et avantages de l'invention sont illustrés par les exemples suivants.

### Exemple 1

### Synthèse du 2-O-naphtalène-sulfonyl-L-tyrosine

### Synthèse de N-formyl-L-tyrosine.

La L-tyrosine (60 g; 0,33 mole) a été dissoute dans de l'acide formique 98% (400 ml) et refroidie à 10°C. L'anhydride acétique (192 ml; 1,73 mole) a été ajoutée en quatre parties pendant cinq minutes tout en maintenant une température de 10°C. Le mélange a ensuite été agité pendant 60 mn à 10°C et maintenu ensuite à température ambiante pendant 30mn. De l'eau (192 ml) a été ajoutée sous agitation à 10-15°C pendant 30 mn et ensuite le mélange a été soumis à évaporation jusqu'à l'obtention d'un résidu sec sous pression réduite à 70°C. Le solide huileux résiduel a été dissout dans un volume minimal d'eau bouillante et ensuite refroidi pour produire de fins cristaux blancs de N-formyl-L-tyrosine(60,6g ; 88%).

### Synthèse de N-formyl-O-(2-naphthalène-sulphonyl)-tyrosine.

### Procédé A

Le N-formyl-L-tyrosine (14,0 g; 0,0668 mole) a été dissout dans de l'acétone (250 ml) et la solution a été diluée avec de l'eau (190 ml). Le pH a été ajusté à 9,7 par addition d'une solution carbonate de sodium saturé et une solution de chlorure de naphthalène-2-sulphonyle (15 g; 0,0668 mole) dans de l'acétone (42 ml) ensuite a été ajouté goutte à goutte pendant 20 mn à température ambiante tout en maintenant le pH à 9,7 par l'addition de solution de carbonate de sodium. Le mélange à été agité pendant 60 mn et mis au repos pendant 12 heures, après quoi l'acétone a été en grande partie évaporée sous une pression réduite à 50°C. La solution résiduelle a été refroidie à 5°C et ensuite extraite avec de l'éther (2x50 ml). La phase aqueuse a ensuite été acidifiée jusqu'à l'obtention d'un pH de 3,5 produisant un précipité blanc dense qui a été recueilli par filtration et séché dans une étuve sous vide à 60°C pour donner un solide blanc (24,57 g; 92%).

### Déformylation

Le N-formyl-O-(2-naphthalène-sulphonyl)-tyrosine (24,57 g; 0,0615 mole) a été remis en suspension dans de l'acétone (105 ml) et ajouté en petite quantité à un mélange de reflux d'acide chlorhydrique concentré (240 ml) et d'eau (240 ml). Le mélange a été chauffé sous reflux pendant 3 heures et ensuite versé dans de l'eau (750ml) sous agitation et refroidissement à 10°C. Le pH a été ajusté à 3,5 et le précipité blanc dense résultant a été récupéré par filtration et séché dans une étuve sous vide à 50°C pour donner une poudre blanche propre (21,25 g; 94%). Le produit pourrait encore être purifié par une solution d'hydroxyde de sodium aqueux et l'addition d'acide. Le rendement du produit sec était de 79%.

Le rendement total obtenu à partir de L-tyrosine est de 64%.

### Exemple 2

### Synthèse du 4-O-tosyl-L-tyrosine

La synthèse du 4-O-toluène-sulfonyl-L-tyrosine a été effectuée selon deux procédés suivants :

### Procédé A.

Le composé a été préparé conformément au procédé décrit dans l'exemple 1 mais en substituant le chlorure de naphathalène-2-sulfonyle par l'équivalent molaire de chlorure de toluène-sulfonyle. Le rendement total en produit était de 56%.

### Procédé B.

Du pentahydrate de sulfate de cuivre (5 g; 20 mmol) a été dissout dans de l'eau (100 ml) sous agitation et une solution d'hydroxyde de potassium (2,25 g; 40 mmole) dans de l'eau a été ajoutée en continu. Après 15 mn, la suspension a été mise à reposer, filtrée et le résidu a été lavé avec une petite quantité d'eau et ensuite partiellement séché pour produire un " résidu bleu ".

La L-tyrosine (7,2 g; 40 mmole) a été remise en suspension dans de l'eau (100 ml) et de l'hydroxyde de potassium aqueux (3,36 g; 60 mmole) a été ajouté. A cette solution chaude a été ajouté de l'hydroxyde de cuivre préparé ci-dessus. La solution résultante bleue foncée du complexe de cuivre a été acidifié soigneusement jusqu'à pH 8. Le précipité pourpre qui s'est formé progressivement a été isolé par filtration, lavé avec de l'eau et du méthanol et séché à l'air pour donner 7,4 g de complexe. Celui-ci a été remis en suspension dans du méthanol (80 ml) et une solution de carbonate de sodium (3,7 g; 35 mmole) a été ajoutée avec agitation. Le complexe de cuivre qui était seulement dissous a nécessité l'utilisation de méthanol pour le clarifier. A la solution agitée, filtrée a été ajouté de façon graduelle une solution de chlorure de 4-toluène-sulphonyle (6,55 g; 35 mmole) dans du méthanol (60 ml). Le pH a été maintenu entre 9 et 11 par l'addition périodique de carbonate de sodium aqueux. Après 2 heures la suspension a été filtrée et le résidu a été lavé avec du méthanol. Les étapes de lavage et de filtration ont été combinées et la suspension a été soumise à évaporation à 45°C pour enlever le méthanol.

Le solide bleu résultant a été décomposé par agitation vigoureuse avec de l'acide chlorhydrique (15 ml) et de la glace (30 g). Le solide vert pale a été enlevé et dissout directement dans de l'eau chaude (100 à 120 ml). Un refroidissement et un ajustement du pH à 7,5 ont produit une grande quantité d'un précipité blanc. Celui-ci a été enlevé par filtration. Le résidu a été redissout dans un volume similaire d'eau chaude contenant de l'EDTA (300 mg) pour enlever les traces d'ions cupriques et le pH a été ajusté de nouveau à 7,5. Le produit a été isolé sous la forme d'un solide blanc argenté après filtration et séchage (6,1 g; 52%).

### Exemple 3

### Synthèse de O-mésitoyl-L-tyrosine

La N-a-t-BOC-L-tyrosine (2,81 g; 10 mmole) a été dissoute dans de la pyridine anhydre (15 ml). A cette solution a été ajouté lentement du chlorure de 2,4-6 triméthylbenzoyle (1,92 g; 10,5 mmole). La température a été maintenue entre 15°C et 25°C. Après agitation pendant 30 mn, le mélange réactionnel a été versé sur de l'eau glacée. Le solide poisseux a été soumis à une extraction dans du dichlorométhane (2x50 ml) et la couche organique a été lavée avec de l'eau (3x50 ml) et séchée sur du sulfate de magnésium anhydre. L'évaporation du solvant à conduit à l'obtention d'un solide vitreux qui cristallisait lentement.

Le produit a été déprotégé en le dissolvant dans une petite quantité d'acétate d'éthyle et par addition d'une solution de chlorure d'hydrogène (3 M) dans de l'acétate d'éthyle (5 ml). La solution agitée graduellement se déposait sous la forme d'un solide blanc. Une précipitation maximale a été obtenue par l'addition de diethyl éther (30 ml).

Le produit a été enlevé par filtration sous vide, lavé avec de l'éther et séché dans un déssiccateur sous vide (2,5 g; 69%).

### Exemple 4

### Synthèse de N-in-mésitylène-2-sulphonyl-L-tryptophane

Le N-a-t-BOC-N-in-mésitylène-2-sulphonyl-L-tryptophane, (1,0 g; 1,5 mmole) a été mis en suspension dans de l'acétate d'éthyle (5 ml) et une solution de chlorure d'hydrogène (3 M) dans de l'acétate d'éthyle (2,5 ml) a été ajoutée à la solution agitée. Un précipité blanc s'est formé rapidement et s'est intensifié en même temps que l'agitation. Après 2 heures, la suspension a été filtrée et le résidu a été lavé avec de l'éther, séché et stocké. Bien que la chromatographie en couche mince montrait seulement un composant (sous visualisation par ultraviolet), le produit était probablement contaminé avec du chlorure de dicyclohexylammonium. Aucun essai n'a été effectué pour enlever ce contaminant et le produit a été utilisé tel que pour être testé. Le rendement était de 0,68 g.

### Exemple 5

### Synthèse de N-im-(4-toluène-sulphonyl)-L-histidine

La N-a-t-BOC-N-im-tosyl-L-histidine (1,0 g; 2,44 mmole) a été dissoute dans une solution de chlorure d'hydrogène (3 M) dans de l'acétate d'éthyle (5 ml). Après agitation pendant 2 heures, le composé a été isolé sous la forme d'un sel d'hydrochlorure comme décrit dans l'exemple 4 et donnait un solide blanc (0,64 g; 76 %).

De la même manière, le O-(2,6-dichlorobenzyl)-L-tyrosine, le N-im-benzyloxycarbonyl-L-histidine, et la Np-benzyloxymethyl-L-histidine ont été préparés par des protections d'intermédiaire commercialement disponibles pour la synthèse des peptides.

Des essais ont été réalisés pour illustrer l'intérêt des composés de formule (I) selon l'invention pour la détection des bactéries du genre *Proteus* sur des milieux gélifiés.

### Exemple 6

Deux milieux ont été préparés selon les techniques habituelles. Ils ont la même composition à l'exception de l'agent de détection utilisé pour la mise en évidence de l'activité enzymatique de type désaminase.

La composition des Milieux I et II pour un litre de milieu final est la suivante :

| | |
|---|---|
| Extrait coeur-cervelle (bioMérieux) | 5 g/l |
| bio-Soyase (bioMérieux) | 5 g/l |
| Tampon Tris (Prolabo) | 1 g/l |
| Phosphate monopotassique (Merck) | 1 g/l |
| Agar (bioMérieux) | 17,5 g/l |
| Citrate de fer ammoniacal (Sigma) | 0,5 g/l |

Le pH des milieux a été ajusté aux environs de 7,2.

Dans le Milieu 1, l'agent de détection de l'activité enzymatique de type désaminase utilisé est l'acide-aminé naturel L-Tyrosine à 1,5 g/l; dans le Milieu II l'agent de détection de l'activité enzymatique de type désaminase utilisé est le 4-O-toluène-sulfonyl-L-tyrosine à 0,5 g/l.

Sur ces deux milieux, 12 souches de bactéries ont été directement cultivées en boîte de Pétri. Les souches provenant de la collection de la demanderesse, appartiennent aux espèces suivantes : *Escherichia coli* (2 souches), *Enterobacter cloacae* (1 souche), *Klebsiella pneumoniae* (1 souche), *Morganella morganii* (2 souche), *Proteus mirabilis* (2 souches), *Proteus vulgaris* (2 souches), *Enterococcus faecalis.* (1 souche), *Staphylococcus saprophyticus.* (1 souche). De plus un mélange d'une souche d'*E*. *coli* et d'une souche de *P. mirabilis* a également été cultivé en boîte de Pétri. Les boîtes ont été incubées à 37°C pendant 48 heures. Les colonies formées ont été examinées visuellement, respectivement après 24 et 48 heures d'incubation selon les interprétations suivantes :
- les colonies brunes correspondent à des souches produisant une désaminase appartenant *a priori* au groupe *Proteus* (ici représentée par les espèces : *M. morganii, P. mirabilis, P. vulgaris*) *;*
- les colonies blanches correspondent aux souches ne produisant pas l'enzyme précitée, elles appartiennent donc à d'autres espèces de bactéries qui seront alors à identifier à l'aide des techniques habituelles ;
- la diffusion du précipité brun autour de la colonie est appréciée selon une échelle semi-quantitative (nulle, faible, forte).

Les résultats sont présentés dans le tableau I ci-après :

**TABLEAU I**

| | | à 24 heures | | | | à 48 heures | | | |
|---|---|---|---|---|---|---|---|---|---|
| | | Colonies | Diffusion | | | Colonies | Diffusion | | |
| Espèces | Milieu | brunes | Nulle | Faible | Forte | brunes | Nulle | Faible | Forte |
| *E. coli* | I | -* | - | - | - | - | - | - | - |
| (2 souches) | II | - | - | - | - | - | - | - | - |
| *E. cloacae* | I | - | - | - | - | - | - | - | - |
| (1 souche) | II | - | - | - | - | - | - | - | - |
| *K. pneumoniae* | I | - | - | - | - | 1 | - | 1 | - |
| (1 souche) | II | - | - | - | - | - | - | - | - |
| *M. morganii* | I | 2 | - | - | 2 | 2 | - | - | 2 |
| (2 souches) | II | 2 | 2 | - | - | 2 | 2 | - | - |
| *P. mirabilis* | I | 2 | - | - | 2 | 2 | - | - | 2 |
| (2 souches) | II | 2 | 2 | - | - | 2 | 2 | - | - |
| *P. vulgaris* | I | 2 | - | - | 2 | 2 | - | - | 2 |
| (2 souches) | II | 2 | 2 | - | - | 2 | 2 | - | - |
| *E. faecalis* | I | - | - | - | - | - | - | - | - |
| (1 souche) | II | - | - | - | - | - | - | - | - |
| *S. saprophyticus* | I | - | - | - | - | - | - | - | - |
| (1 souche) | II | - | - | - | - | - | - | - | - |

| | | | | | | | | | |
|---|---|---|---|---|---|---|---|---|---|
| * : nombre de souches, "-" = 0 | | | | | | | | | |

Comme cela ressort du tableau I ci-dessus, le procédé selon l'invention permet une détection des bactéries du groupe *Proteus.* En effet pour la culture sur le Milieu I, on ne distingue qu'un type de colonies brun-translucide sur fond d'un milieu orangé, alors que sur le milieu II selon l'invention il ressort deux types de colonies : les unes brunes et les autres translucides sur fond d'un milieu incolore à brun au voisinage des colonies colorées.

### Exemple 7

Bien que le procédé selon l'invention soit plus avantageusement adapté à la détection d'une activité enzymatique de type désaminase en milieu gélifié ou sur support solide, il peut également être utilisé en milieu liquide.

Deux milieux ont été préparés selon les techniques habituelles. Ils ont la même composition à l'exception de l'agent de détection utilisé pour révéler l'activité enzymatique de type désaminase.

La composition des Milieux III et IV pour un litre de milieu final est la suivante :

| | |
|---|---|
| Extrait coeur-cervelle (bioMérieux) | 5 g/l |
| bio-Soyase (bioMérieux) | 5 g/l |
| Tampon Tris (Sigma) | 1 g/l |
| Phosphate monopotassique (Merck) | 1 g/l |
| Citrate de fer ammoniacal (Sigma) | 0,5 g/l |

Le pH des milieux a été ajusté aux environs de 7,2.

Dans le Milieu III, l'agent de détection de l'activité enzymatique de type désaminase utilisé est l'acide-aminé naturel L-histidine à 1,5 g/l ; dans le Milieu IV l'agent de détection de l'activité enzymatique de type désaminase utilisé est le N-toluène-sulfonyle-L-histidine à 0,5g/l. Les milieux ont été répartis stérilement en tube à essai.

Ces deux milieux ont été inoculés avec les 12 souches de bactéries décrites dans l'exemple 6. Les tubes ont été incubés pendant 48 heures à 37°C et examinés visuellement après 48 heures selon l'interprétation suivante :
- le brunissement du tube traduit la présence d'une activité enzymatique de type désaminase et donc d'une bactérie du groupe *Proteus,*
- l'absence de brunissement signifie l'absence de bactérie du groupe *Proteus.*

Les résultats sont présentés dans le tableau II ci-après :

**TABLEAU II**

| | Brunissement du milieu | | | |
|---|---|---|---|---|
| | à 48 heures | | | |
| Espèces | Milieu | Fort | Faible | Nul |
| *E. coli* | III | - | 2 | - |
| (2 souches) | IV | - | - | 2 |
| *E. cloacae* | III | - | 1 | - |
| (1 souche) | IV | - | - | 1 |
| *K. pneumoniae* | III | - | 1 | - |
| (1 souche) | IV | - | - | 1 |
| *M. morganii* | III | 1 | 1 | - |
| (2 souches) | IV | 2 | - | - |
| *P. mirabilis* | III | 2 | - | - |
| (2 souches) | IV | 2 | - | - |
| *P. vulgaris* | III | 2 | - | - |
| (2 souches) | IV | 2 | - | - |
| *E. faecalis* | III | 1 | - | - |
| (1 souche) | IV | - | - | 1 |
| *S. saprophyticus* | III | - | - | 1 |
| (1 souche) | IV | - | - | 1 |

| | | | | |
|---|---|---|---|---|
| : nombre de souches, "-" = 0 | | | | |

Comme cela ressort du tableau II ci-dessus, en milieu liquide, l'agent de détection selon l'invention permet comme la L-histidine de différencier les bactéries appartenant au groupe *Proteus* de celles n'y appartenant pas, avec une sensibilité et une spécificité élevée après 48 heures.

### Exemple 8

Des essais ont également été entrepris pour tester la possibilité de détecter simultanément une activité enzymatique de type désaminase selon l'invention ainsi qu'une autre activité enzymatique.

Pour cela trois milieux ont été préparés selon les techniques habituelles. Le premier (Milieu V) correspond au Milieu II de l'exemple 6 dans lequel le 4-O-toluène-sulfonyle-L-tyrosine a été remplacé par du 4-O-dansyl-L-tyrosine à la même concentration, le deuxième (Milieu VI) correspond à ce même milieu mais avec du 5-bromo-4-chloro-3-indolyl-β-D-glucoside à 0,1 g/l à la place du substrat de désaminase et le troisième (Milieu VII) correspond au milieu VI additionné de 4-O-dansyl-L-tyrosine à 0,5 g/l.

Sur ces trois milieux, les 12 souches de bactéries décrites dans l'exemple 6 ont été directement cultivées en boîte de Pétri. De plus un mélange d'une souche d'*K. pneumoniae* et d'une souche de *P. mirabilis* a également été cultivé en boîte de Pétri. Les boîtes ont été incubées à 37°C pendant 24 heures. Les colonies formées ont été examinées visuellement, après 24 heures d'incubation selon les interprétations suivantes :
- les colonies brunes correspondent à des souches produisant une désaminase appartenant *a priori* au groupe *Proteus* (ici représentée par les espèces : *M. morganii, P. mirabilis, P. vulgaris*) ;
- les colonies bleues correspondent aux souches exprimant une activité β-D-glucosidase et hydrolysant le 5-bromo-4-chloro-3-indolyl-β-D-glucoside ;
- les colonies blanches correspondent aux souches ne produisant pas les enzymes précitées.

Les résultats sont présentés dans le tableau III ci-après :

**TABLEAU III**

| | | 24 Heures | | | |
|---|---|---|---|---|---|
| | | Coloration des colonies | | | |
| Espèces | Milieu | Incolore | Bleue | Brun-Bleu | Brune |
| | V | 2 | - | - | - |
| *E. coli* | VI | 2 | - | - | - |
| (2 souches) | VII | 1 | - | - | 1 |
| | V | 1- | - | - | - |
| *E. cloacae* | VI | - | 1 | - | - |
| (1 souche) | VII | - | 1 | - | - |
| | V | 1 | - | - | - |
| *K. pneumoniae* | VI | - | 1 | - | - |
| (1 souche) | VII | - | 1 | - | - |
| | V | - | - | - | 2 |
| *M. morganii* | VI | 2 | - | - | - |
| (2 souches) | VII | - | - | - | 2 |
| | V | - | - | - | 2 |
| *P. mirabilis* | VI | 2 | - | - | - |
| (2 souches) | VII | - | - | - | 2 |
| | V | - | - | - | 2 |
| *P. vulgaris* | VI | - | 2 | - | - |
| (2 souches) | VII | - | - | 2 | - |
| | V | 1 | - | - | - |
| *E. faecalis* | VI | - | 1 | - | - |
| (1 souche) | VII | - | 1 | - | - |
| | V | 1 | - | - | - |
| *S. saprophyticus* | VI | 1 | - | - | - |
| (1 souche) | VII | 1 | - | - | - |

| | | | | | |
|---|---|---|---|---|---|
| * : nombre de souches, "-" = 0 | | | | | |

Comme cela ressort du tableau III ci-dessus, le procédé selon l'invention permet une détection des bactéries du groupe *Proteus* et n'interfère pas avec la détection des colonies β-glucosidase, y compris en mélange. En effet pour la culture du mélange de *K. pneumoniae* et de *P. mirabilis,* sur le Milieu VII, on distingue parfaitement les deux types de colonies, les unes brunes et les autres bleues. Par ailleurs si une bactérie exprime simultanément les deux activités enzymatiques (β-glucosidase et désaminase) cela se traduit par la formation de colonies ayant une couleur résultant d'un mélange de bleu et de brun, comme c'est le cas pour une souche de *P. vulgaris* sur le milieu VII.

### Exemple 9

Le procédé et composés selon l'invention sont notamment parfaitement adaptés à l'isolement et à l'identification des micro-organismes urinaires. Le milieu VIII a été développé pour cette application, il a la composition suivante :

| | |
|---|---|
| Extrait coeur-cervelle (bioMérieux) | 5 g/l |
| bio-Soyase (bioMérieux) | 5 g/l |
| Tampon Tris (Sigma) | 1 g/l |
| Phosphate monopotassique (Merck) | 1 g/l |
| Citrate de fer ammoniacal (Sigma) | 0,5 g/l |
| 4-O-toluène-sulfonyle-L-tyrosine | 0,4 g/l |
| 5-bromo-4-chloro-3-indolyl-β-D-glucoside (Biosynth) | 0,06 g/l |
| 6-chloro-3-indolyl-β-D-glucuronide (Biosynth) | 0,25 g/l |
| Méthyl-β-D-glucuronide (Sigma) | 0,1 g/l |

Le pH des milieux a été ajusté aux environs de 7,2.

Sur ce milieu, 25 souches de bactéries ont été directement cultivées en boîte de Pétri. Les souches provenant de la collection de la demanderesse, appartiennent aux espèces suivantes : *E. coli* (3 souches), *Citrobacter diversus* (2 souches), *E. cloacae* (2 souches), *K. pneumoniae* (2 souches), *M. morganii* (2 souches), *P. mirabilis* (3 souches), *P*. *vulgaris* (2 souches), *E. faecalis. (2* souches), *Staphylococcus aureus* (2 souches), *S. saprophyticus.* (3 souches), *Candida albicans* (2 souches). De plus un mélange d'une souche d'*E*. *coli,* d'une souche de *K. pneumoniae* et d'une souche de *P. mirabilis* a également été cultivé en boîte de Pétri. Les boîtes ont été incubées à 37°C pendant 48 heures. Les colonies formées ont été examinées visuellement, respectivement après 24 et 48 heures d'incubation selon les interprétations suivantes :
- les colonies brunes correspondent à des souches produisant une désaminase appartenant *a priori* au groupe *Proteus* (ici représentée par les espèces : *M. morganii, P. mirabilis, P. vulgaris*) ;
- les colonies rose-tyrien correspondent à des souches produisant une β-D-glucuronidase appartenant *a priori* à l'espèce *E*. *coli*
- les colonies bleues correspondent à des souches produisant une β-D-glucosidase, appartenant *a priori* soit au groupe des *KlebsiellalEnterobacterlSerratia* soit au genre *Enterococcus ;*
- les colonies blanches correspondent aux souches ne produisant pas les enzymes précitées, elles appartiennent donc à d'autres espèces de bactéries ou de levures qui seront alors à identifier à l'aide des techniques habituelles.

Les résultats sont présentés dans le tableau IV ci-après :

**TABLEAU IV**

| | | | | | | | 48 Heures | | | | |
|---|---|---|---|---|---|---|---|---|---|---|---|
| | | Coloration des colonies | | | | | Coloration des colonies | | | | |
| Espèces | Milieu | Incolore | Brune | Gris-Vert | Bleu | Rose-tyrien | Incolore | Brune | Gris-Vert | Bleue | Rose-tyrien |
| *E. coli* | VIII | - | - | - | - | 3 | - | - | - | - | 3 |
| (3 souches) | | | | | | | | | | | |
| *C. diversus* | VIII | - | - | - | 2 | - | - | - | - | 2 | - |
| (2 souches) | | | | | | | | | | | |
| *E. cloacae* | VIII | - | - | - | 2 | - | - | - | - | 2 | - |
| (2 souches) | | | | | | | | | | | |
| *K. pneumoniae* | VIII | - | - | - | 2 | - | - | - | - | 2 | - |
| (2 souches) | | | | | | | | | | | |
| *M. morganii* | VIII | - | 2 | - | - | - | - | 2 | - | - | - |
| (2 souches) | | | | | | | | | | | |
| *P. mirabilis* | VIII | - | 3 | - | 3 | - | - | 3 | - | - | - |
| (3 souches) | | | | | | | | | | | |
| *P. vulgaris* | VIII | - | - | 2 | - | - | - | - | 2 | - | - |
| (2 souches) | | | | | | | | | | | |
| *E. faecalis* | VIII | - | - | - | 2 | - | - | - | - | 2 | - |
| (2 souches) | | | | | | | | | | | |
| *S. aureus* | VIII | 2 | - | - | - | - | 2 | - | - | - | - |
| (2 souches) | | | | | | | | | | | |
| *S. saprophyticus* | VIII | 2 | - | - | - | - | 2 | - | - | - | - |
| (3 souches) | | | | | | | | | | | |
| *C. albicans* | VIII | 2 | - | - | - | - | 2 | - | - | - | - |
| (2 souches) | | | | | | | | | | | |
| Mélange plurimicrobien | VIII | - | 1 | - | 1 | 1 | - | 1 | - | 1 | 1 |
| (3 souches) | | | | | | | | | | | |

| | | | | | | | | | | | |
|---|---|---|---|---|---|---|---|---|---|---|---|
| * : nombre de souches, "-" = 0 | | | | | | | | | | | |

Comme cela ressort du tableau IV ci-dessus, le procédé selon l'invention permet une détection des principales bactéries urinaires y compris en mélange. En effet il est possible sur le milieu VIII de détecter quatre types de colonies différents (incolore, rose-tyrien, bleu et brun), même dans le cas de culture plurimicrobienne. Un tel milieu est particulièrement utile puisqu'il permet, dès l'isolement, de détecter la très grande majorité des micro-organismes urinaires simplifiant de ce fait et l'identification, et l'analyse des échantillons les plus complexes, apportant donc simultanément un gain dans la qualité de l'analyse et dans son coût.

## Revendications

1. Procédé de détection et d'identification et/ou quantification d'une activité enzymatique de type désaminase de micro-organisme, selon lequel on met en contact un inoculum susceptible de contenir un micro-organisme ayant une activité désaminase avec un milieu de culture pour micro-organismes,
**caractérisé en ce que** le milieu de culture comprend au moins un agent de détection permettant de mettre en évidence, par formation d'un produit coloré avec un agent révélateur, une activité enzymatique de type désaminase,
ledit agent de détection étant un L-acide aminé de formule (I) générale suivante: 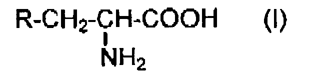 dans laquelle:
- R représente un radical d'acide-aminé cyclique, substitué par 1 à 3 groupements X, identiques ou différents,
- X représente un groupement limitant la diffusion de l'α-céto acide produit par la désamination de l'acide-aminé cyclique, et X est choisi parmi le méthyle, le benzyle, le carboxybenzyle, le dansyle, le naphtalène-sulfonyle, le tosyl-sulfonyle, le mésitylène-sulfonyle.

2. Procédé selon la revendication 1, **caractérisé en ce que** l'agent révélateur est un sel de cation.

3. Procédé selon la revendication 1, **caractérisé en ce que** l'agent révélateur est ajouté au milieu de culture en même temps que l'agent de détection.

4. Procédé selon la revendication 1, **caractérisé en ce que** l'agent révélateur est ajouté au milieu de culture après la mise en culture des micro-organismes.

5. Procédé selon la revendication 1, **caractérisé en ce** les micro-organismes détectés et identifiés et/ou quantifiés par l'activité enzymatique de type désaminase appartiennent au genre *Proteus*.

6. Procédé selon la revendication 1, **caractérisé en ce qu'**on ajoute également audit milieu de culture au moins un autre agent de détection permettant de mettre en évidence, par formation d'un produit coloré ou fluorescent, une activité enzymatique différente de celle mise en évidence par le composé de formule générale (1),

7. Agent de détection consistant en le O-(2-naphtalène-sulfonyl)-tyrosine.

8. Utilisation d'un composé ayant la formule générale (I) suivante: 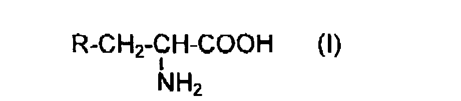 dans laquelle:
- R représente un radical d'acide-aminé cyclique, substitué par un groupement X,
- X est choisi parmi le méthyle, le benzyle, le carboxybenzoyle, le dansyl, le naphtalène-sulfonyle, le toluène sulfonyle, et le mésitylène sulfonyle,
pour détecter et identifier et/ou quantifier une activité enzymatique de type désaminase de micro-organisme.

9. Utilisation selon la revendication 8, **caractérisée en ce que** le composé est le O-(2-naphtalène-sulfonyl)-tyrosine.

10. Utilisation selon la revendication 8, **caractérisée en ce que** le composé est le 4-O-toluène-sulfonyl-L-tyrosine.

11. Utilisation selon la revendication 8, **caractérisée en ce que** le composé est le N-toluène-sulfonyl-L-histidine.

12. Procédé de préparation des composés selon la revendication 8, comprenant les étapes suivantes:
(a) formylation du reste R,
(b) addition d'un sel de X sur le reste R formylé selon (a),
(c) déformylation du reste R substitué selon (b).

13. Milieu de culture de micro-organismes comprenant, outre les ingrédients nécessaires à la culture desdits micro-organismes, au moins un agent de détection ou composé selon l'une quelconque des revendications 7 à 11.

14. Milieu de culture selon la revendication 13, **caractérisé en ce que** la concentration pondérale du ou des agents de détection est comprise entre 0,025 et 5 g/l de milieu de culture.

15. Milieu de culture selon les revendications 13 et 14, **caractérisé en ce que** la concentration pondérale du ou des agents de détection ou composés est comprise entre 0,1 et 2 g/l, de préférence entre 0,3 et 0,6 g/l.

16. Milieu de culture selon la revendication 13, **caractérisé en ce qu'**il comprend également un agent révélateur, de préférence un sel de cation, par exemple du citrate de fer ammoniacal.

17. Milieu de culture selon la revendication 13, **caractérisé en ce qu'**il est sous forme gélosé.

18. Milieu de culture selon les revendications 13 à 17, **caractérisé en ce qu'**il comprend également au moins un autre agent de détection permettant de mettre en évidence, par formation d'un produit coloré ou fluorescent, une activité enzymatique différente de celle mise en évidence par le composé de formule générale (I).

## Claims

1. Method for detecting and identifying and/or quantifying an enzymatic activity such as deaminase activity of a microorganism, according to which an inoculum which is capable of containing a microorganism with a deaminase activity is brought into contact with a culture medium for microorganisms,
**characterized in that** the culture medium comprises at least one detection agent for demonstrating, by forming a coloured product with a revealing agent, an enzymatic activity such as deaminase activity;
said detection agent being an L-amino acid of following general formula (I): 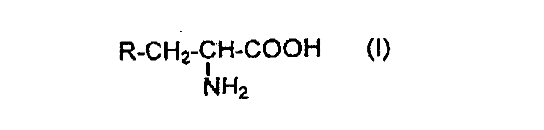 in which:
- R represents a cyclic amino acid radical, substituted with 1 to 3 groups X, which are identical or different,
- X represents a group which limits the diffusion of the α-keto acid produced by the deamination of the cyclic amino acid, and X is chosen from methyl, benzyl, carboxybenzyl, dansyl, naphthalenesulphonyl, tosylsulphonyl and mesitylenesulphonyl.

2. Method according to Claim 1, **characterized in that** the revealing agent is a cation salt.

3. Method according to Claim 1, **characterized in that** the revealing agent is added to the culture medium at the same time as the detection agent.

4. Method according to Claim 1, **characterized in that** the revealing agent is added to the culture medium after culturing the microorganisms.

5. Method according to Claim 1, **characterized in that** the microorganisms which are detected and identified and/or quantified by enzymatic activity such as deaminase activity belong to the group *Proteus.*

6. Method according to Claim 1, **characterized in that** at least one other detection agent for demonstrating, by forming a coloured or fluorescent product, an enzymatic activity which is different from that demonstrated by the compound of general formula (I) is also added to said culture medium.

7. Detection agent consisting of O-(2-naphthalenesulphonyl)tyrosine.

8. Use of a compound having the following general formula (I): 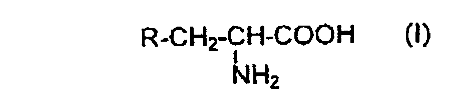 in which:
- R represents a cyclic amino acid radical, substituted with a group X,
- X is chosen from methyl, benzyl, carboxybenzoyl, dansyl, naphthalenesulphonyl, toluenesulphonyl and mesitylenesulphonyl,
for detecting and identifying and/or quantifying an enzymatic activity such as deaminase activity of a microorganism.

9. Use according to Claim 8, **characterized in that** the compound is O-(2-naphthalenesulphonyl)tyrosine.

10. Use according to Claim 8, **characterized in that** the compound is 4-0-toluenesulphonyl-L-tyrosine.

11. Use according to Claim 8, **characterized in that** the compound is N-toluenesulphonyl-L-histidine.

12. Method for preparing the compounds according to Claim 8, comprising the following steps:
(a) formylation of the residue R,
(b) addition of a salt of X onto the residue R formylated according to (a),
(c) deformylation of the residue R substituted according to (b).

13. Culture medium for microorganisms, comprising, besides the ingredients required for culturing said microorganisms, at least one detection agent or compound according to any one of Claims 7 to 11.

14. Culture medium according to Claim 13, **characterized in that** the weight concentration of the detection agent(s) is between 0.025 and 5 g/l of culture medium.

15. Culture medium according to Claims 13 and 14, **characterized in that** the weight concentration of the detection agent(s) or compound(s) is between 0.1 and 2 g/l, preferably between 0.3 and 0.6 g/l.

16. Culture medium according to Claim 13, **characterized in that** it also comprises a revealing agent, preferably a cation salt, for example ammoniacal iron citrate.

17. Culture medium according to Claim 13, **characterized in that** it is in a gelled form.

18. Culture medium according to Claims 13 to 17, **characterized in that** it also comprises at least one other detection agent for demonstrating, by forming a coloured or fluorescent product, an enzymatic activity which is different from that demonstrated by the compound of general formula (I).

## Patentansprüche

1. Verfahren zum Nachweisen und Identifizieren und/oder Quantifizieren einer Mikroorganismen-Enzymaktivität des Desaminasetyps, bei dem man ein Inokulum, das einen Mikroorganismus mit Desaminaseaktivität enthalten kann, mit einem Kulturmedium für Mikroorganismen in Kontakt bringt,
**dadurch gekennzeichnet, daß** das Kulturmedium mindestens ein Nachweismittel enthält, mit dem man durch Bildung eines gefärbten Produkts mittels eines Entwicklers eine Enzymaktivität des Desaminasetyps nachweisen kann,
wobei es sich bei dem Nachweismittel um eine L-Aminosäure der folgenden allgemeinen Formel (I) handelt: 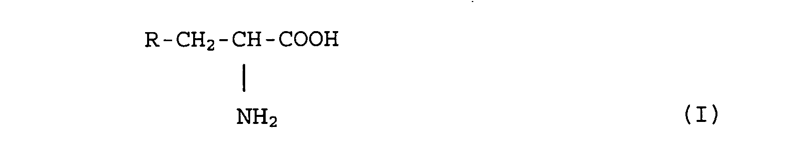 in der
- R einen zyklischen Aminosäurerest bedeutet, der durch 1 bis 3 gleiche oder verschiedene Gruppen X substituiert ist,
- X eine Gruppe bedeutet, die die Diffusion der durch Desaminierung der zyklischen Aminosäure erzeugten α-Ketosäure limitiert, und X aus der Reihe Methyl, Benzyl, Carboxybenzyl, Dansyl, Naphthalinsulfonyl, Tosylsulfonyl und Mesitylensulfonyl stammt.

2. Verfahren nach Anspruch 1, **dadurch gekennzeichnet, daß** es sich bei dem Entwickler um ein Kationensalz handelt.

3. Verfahren nach Anspruch 1, **dadurch gekennzeichnet, daß** der Entwickler gleichzeitig mit dem Nachweismittel zu dem Kulturmedium gegeben wird.

4. Verfahren nach Anspruch 1, **dadurch gekennzeichnet, daß** der Entwickler nach Kultivierung der Mikroorganismen zu dem Kulturmedium gegeben wird.

5. Verfahren nach Anspruch 1, **dadurch gekennzeichnet, daß** die Mikroorganismen, die durch die Enzymaktivität des Desaminasetyps nachgewiesen und identifiziert und/oder quantifiziert werden, zur Gattung *Proteus* gehören.

6. Verfahren nach Anspruch 1, **dadurch gekennzeichnet, daß** man noch mindestens ein weiteres Nachweismittel, mit dem durch Bildung eines gefärbten oder fluoreszierenden Produkts eine Enzymaktivität, die sich von der mit der Verbindung der allgemeinen Formel (I) nachgewiesenen Enzymaktivität unterscheidet, nachgewiesen werden kann, zugibt.

7. Nachweismittel, das aus O-(2-Naphthalinsulfonyl)tyrosin besteht.

8. Verwendung einer Verbindung der folgenden allgemeinen Formel (I): 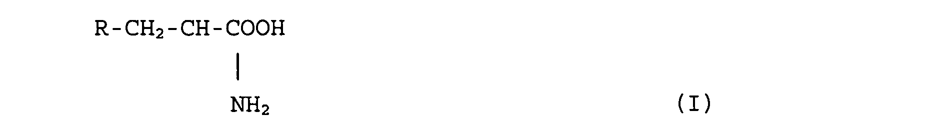 in der
- R einen zyklischen Aminosäurerest bedeutet, der durch eine Gruppe X substituiert ist,
- X aus der Reihe Methyl, Benzyl, Carboxybenzoyl, Dansyl, Naphthalinsulfonyl, Toluolsulfonyl und Mesitylensulfonyl stammt,
zum Nachweisen und Identifizieren und/oder Quantifizieren einer Mikroorganismen-Enzymaktivität des Desaminasetyps.

9. Verwendung nach Anspruch 8, **dadurch gekennzeichnet, daß** es sich bei der Verbindung um O-(2-Naphthalinsulfonyl)tyrosin handelt.

10. Verwendung nach Anspruch 8, **dadurch gekennzeichnet, daß** es sich bei der Verbindung um 4-O-Toluolsulfonyl-L-tyrosin handelt.

11. Verwendung nach Anspruch 8, **dadurch gekennzeichnet, daß** es sich bei der Verbindung um N-Toluolsulfonyl-L-histidin handelt.

12. Verfahren zur Herstellung von Verbindungen nach Anspruch 8, umfassend die folgenden Schritte:
(a) Formylierung des Rests R,
(b) Addition des formylierten Rests nach (a) an ein Salz von X,
(c) Deformylierung des gemäß (b) substituierten Rests R.

13. Kulturmedium für Mikroorganismen, das zusätzlich zu den für die Kultur dieser Mikroorganismen erforderlichen Bestandteile noch mindestens ein Nachweismittel oder eine Verbindung nach einem der Ansprüche 7 bis 11 umfaßt.

14. Kulturmedium nach Anspruch 13, **dadurch gekennzeichnet, daß** die Gewichtskonzentration des bzw. der Nachweismittel(s) zwischen 0,025 und 5 g/l des Kulturmediums beträgt.

15. Kulturmedium nach den Ansprüchen 13 und 14, **dadurch gekennzeichnet, daß** die Gewichtskonzentration des bzw. der Nachweismittel(s) oder Verbindung(en) zwischen 0,1 und 2 g/l, vorzugsweise zwischen 0,3 und 0,6 g/l, beträgt.

16. Kulturmedium nach Anspruch 13, **dadurch gekennzeichnet, daß** es weiterhin einen Entwickler, vorzugsweise ein Kationensalz, zum Beispiel Eisenammoniumcitrat, enthält.

17. Kulturmedium nach Anspruch 13, **dadurch gekennzeichnet, daß** es in mit Agar verfestigter Form vorliegt.

18. Kulturmedium nach den Ansprüchen 13 bis 17, **dadurch gekennzeichnet, daß** es noch mindestens ein weiteres Nachweismittel, mit dem durch Bildung eines gefärbten oder fluoreszierenden Produkts eine Enzymaktivität, die sich von der mit der Verbindung der allgemeinen Formel (I) nachgewiesenen Enzymaktivität unterscheidet, nachgewiesen werden kann, enthält.
